# EUROPEAN PATENT APPLICATION

(11) **EP 4 268 806 A1**
(43) Date of publication of application: **01.11.2023**
(21) Application number: 23169693.1
(22) Date of filing: 25.04.2023
(51) Int. Cl.: A61K 9/20, A61K 31/41, A61K 31/216

(54) **A TABLET COMPRISING SACUBITRIL AND VALSARTAN PROCESSED WITH DRY GRANULATION**

(30) Priority: 26.04.2022 TR 202206846
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); SEZER, Dilay, Istanbul (TR); ERDOGAN, Akif, Istanbul (TR)
(74) Representative: Sevinç, Erkan

(57) **Abstract**

The present invention relates to a film coated tablet comprising intragranular composition comprising sacubitril sodium, valsartan disodium and an extragranular composition, wherein the tablet is obtained by dry granulation.

## Description

### Field of the Invention

The present invention relates to a film coated tablet comprising intragranular composition comprising sacubitril sodium, valsartan disodium and an extragranular composition, wherein the tablet is obtained by dry granulation.

### Background of the Invention

Sacubitril is an antihypertensive drug used in combination with valsartan. Its chemical designation is 4-(((2S,4R)-1-([1,1'-biphenyl]-4-yl)-5-ethoxy-4-methyl-5-oxopentan-2-yl)amino)-4-oxobutanoic acid with the following chemical structure of Formula I.

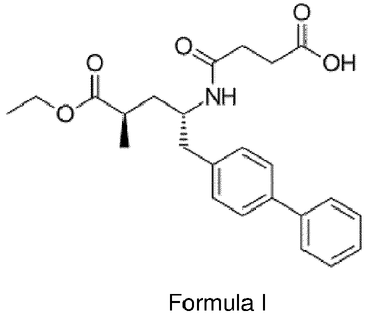

Valsartan is a specific angiotensin II receptor antagonist, which selectively and competitively blocks AT1-receptors and mediates the vasopressor and aldosterone effects of angiotensin II. Its chemical designation is N-(1-oxopentyl)-N-[[2'-(1H-tetrazol-5-yl)-[1,1'-biphenyl]-4-yl]methyl]-L-valine with the following chemical structure of Formula II.

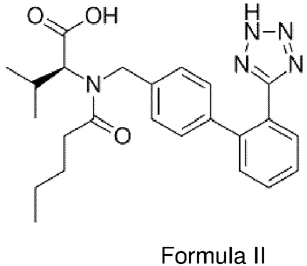

A complex comprising valsartan, which is an angiotensin receptor antagonist, and Sacubitril, which is a neurolysin inhibitor, has been approved by US Food and Drug Administration (FDA) under the trade name ENTRESTO^{®} by Novartis for the treatment of heart failure with reduced ejection fraction. Entresto^{®} is supplied for oral administration as immediate release film-coated tablets in three strengths: 24/26, 49/51 and 97/103 mg, containing respectively 24.3 mg sacubitril/25.7 mg valsartan, 48.6 mg sacubitril/51.4 mg valsartan and 97.2 mg sacubitril/102.8 mg valsartan.

US 8101659 and US 8404744 discloses a composition comprising Valsartan and Sacubitril which are administered in combination in 1:1 ratio.

US 8796331 discloses a method of treating hypertension and heart failure by administering a combination of Valsartan and Sacubitril administered in one unit dose form or in two separate unit dose forms.

WO 2017/012600 discloses a tablet containing a physical mixture of sacubitril or a pharmaceutically acceptable salt thereof and valsartan or a pharmaceutically acceptable salt thereof that can be prepared by direct compression, dry-granulation or wet-granulation.

Valsartan disodium and sacubitril sodium cannot be easily processed due to their poor flowability. Moreover, the amorphous forms of valsartan disodium and sacubitril sodium are very hygroscopic solids. These substances become deliquescent and sticky when exposed to air humidity. Therefore, formulation with these agents and their processing steps are crucial for tablet stability and dissolution.

In prior art, there are also several patents which disclose sacubitril sodium and valsartan disodium in oral pharmaceutical dosage forms. However, despite problems of the poor flowability and stability of sacubitril sodium and valsartan disodium an effective formulation and method has not been disclosed.

There still remains a need in the art to provide an improved a film coated tablet comprising sacubitril sodium and valsartan disodium having high solubility, excellent physicochemical properties and accordingly a high bioavailability and a long-term stability.

### Detailed Description of the Invention

The main object of the present invention is to provide a film coated tablet comprising sacubitril sodium and valsartan disodium with having desired level of dissolution rate and excellent physicochemical properties, such as flowability, compressibility, homogeneity, and content uniformity which overcomes the above-described problems in the prior art and have additive advantages over them.

Another object of the present invention is to provide a film coated tablet comprising sacubitril sodium and valsartan disodium with high stability.

Another object of the present invention is to provide a process for preparing a film coated tablet composition comprising sacubitril sodium and valsartan disodium with high stability. The process is a simple, rapid, cost effective, time-saving, and industrially convenient method. The process is dry granulation.

According to this embodiment of the present invention, a film coated tablet comprises;
- an intragranular composition comprising sacubitril sodium, valsartan disodium and at least one disintegrant,
- an extragranular composition comprising at least one disintegrant,
Wherein the amount of the disintegrants is 4.0% to 15.0% by weight of the total core tablet and the tablet is obtained by dry granulation.

The ratio of active substances in the total tablet is quite high. Due to this reason as well as the characteristic features of the active ingredients, fluidity and homogeneity problems arise to. These problems were overcome with this preferred disintegrant ratio and dry granulation.

The tablet is prepared with dry granulation. The dry granulation is simple and low-cost method. Since valsartan disodium and sacubitril sodium are very hygroscopic solids, this method and the formulation prepared with the excipients mentioned below provided stability.

According to this embodiment of the present invention, the amount of sacubitril sodium is 20.0% to 30.0% by weight of the total core tablet.

According to this embodiment of the present invention, the amount of valsartan disodium is 22.0% to 35.0% by weight of the total core tablet.

Suitable disintegrants are selected from the group comprising crospovidone, sodium starch glycolate, croscarmellose sodium, carboxymethyl cellulose, sodium alginate, corn starch, sodium glycine carbonate or mixtures thereof.

According to this embodiment of the present invention, the disintegrant is crospovidone.

According to this embodiment of the present invention, crospovidone is used both an intragranular composition and an extragranular composition. This provides the desired dissolution profile. Furthermore, this helps to achieve an effective and an easy process.

According to this embodiment of the present invention, the amount of disintegrant is 5.0% to 10.0% by weight of the total core tablet.

According to this embodiment of the present invention, the dry granulation comprises the following steps;
- Preparing intragranular composition comprising sacubitril sodium, valsartan disodium and at least one pharmaceutically acceptable excipient,
- Preparing extragranular composition comprising at least one pharmaceutically acceptable excipient,
- Compressing the total mixture, and obtained core tablets,
- Coating the core tablet with a film coating,
wherein same excipients have been used for each composition. So, this helps to achieve an effective and an easy process.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agents and the excipients.

According to this embodiment of the present invention, each composition further comprises the pharmaceutically acceptable excipients which are selected from the group comprising fillers, binders, disintegrants, lubricants, glidants or mixtures thereof.

Suitable fillers are selected from the group comprising microcrystalline cellulose, pregelatinized starch, mannitol, dicalcium phosphate, lactose monohydrate, calcium carbonate, anhydrous lactose, calcium phosphate, calcium phosphate dehydrate, neutral pellets, cellulose acetate, ethylcellulose, polydextrose, polymethacrylates, sodium alginate, sodium chloride, sugar sphericals or mixtures thereof.

According to this embodiment of the present invention, the filler is microcrystalline cellulose. The filler helps to provide the desired dissolution profile. Also, it helps to provide the desired stability.

According to this embodiment of the present invention, the amount of filler is 15.0% to 25.0% by weight of the total core tablet.

The identification of critical binder attributes in the invention enables rational and efficient binder selection of well soluble and poorly soluble formulations. Binder addition is especially valuable for a poorly soluble formulation. Binder can lead to processing problems such as rapid over granulation, tablet hardness increases and dissolution concert diminish.

Suitable binders are selected from the group comprising low-substituted hydroxypropyl cellulose, hydroxypropyl cellulose, carboxymethylcellulose sodium, cellulose acetate phthalate, ethyl cellulose, glyceryl behenate, hydroxyethyl cellulose, hydroxyethylmethyl cellulose, hydroxypropyl starch, hypromellose, magnesium aluminum silicate, methylcellulose, polyethylene oxide, polymethacrylates, aluminum hydroxide, polyoxyethilene-alkyl ethers, pullulan or mixtures thereof.

According to this embodiment of the present invention, the binder is low-substituted hydroxypropyl cellulose. Especially, using low-substituted hydroxypropyl cellulose provides the desired dissolution profile and the desired stability.

According to this embodiment of the present invention, the amount of binders is 8.0% to 15.0% by weight of the total core tablet.

Suitable lubricants are selected from the group comprising talc, magnesium stearate, sodium stearyl fumarate, calcium stearate, zinc stearate, magnesium lauryl sulfate, sodium oleate, polyethylene glycol, sodium lauryl sulphate or mixtures thereof.

According to this embodiment of the present invention, the lubricant is magnesium stearate or talc or mixtures thereof. It improves the powder processing properties. Also, it enhances powder flow by reducing the inter-particle friction.

According to this embodiment of the present invention, the amount of lubricant is 0.8% to 11.5% by weight of the total composition.

Suitable glidants are selected from the group comprising silica colloidal anhydrous, silicon dioxide, aluminum silicate or mixtures thereof.

According to this embodiment of the present invention, the glidant is silica colloidal anhydrous.

According to this embodiment of the present invention, the amount of glidant is 0.2% to 5.0% by weight of the total composition.

According to this embodiment of the present invention, the film coated tablet comprises;
- Sacubitril sodium
- Valsartan disodium
- Low-substituted hydroxypropylcellulose
- Microcrystalline cellulose
- Crospovidone
- Silica colloidal anhydrous

According to this embodiment of the present invention, the film coated tablet comprises;
- Sacubitril Sodium
- Valsartan Disodium
- Low-substituted hydroxypropylcellulose
- Microcrystalline cellulose
- Crospovidone
- Silica colloidal anhydrous
- Magnesium stearate
- Talc

According to this embodiment of the present invention, a process for preparing a film coated tablet comprising sacubitril sodium and valsartan disodium comprises the following steps;
- Sieving sacubitril sodium and at least one lubricant and then mixing,
- Sieving at least one filler, at least one binder and at least one disintegrant and then mixing,
- Adding valsartan disodium and mixing,
- Sieving at least one glidant and then adding and mixing,
- Sieving at least one lubricant and then adding and mixing, and obtained intragranular composition,
- Compacting the intragranular composition,
- Sieving at least one disintegrant and adding into the intragranular composition and then mixing,
- Sieving at least one glidant and then adding and mixing,
- Sieving at least one lubricant and then adding and mixing,
- Adding at least one lubricant and mixing,
- Compressing the total mixture, and obtained core tablets,
- Coating the core tablet with a film coating.

The present invention relates to a process for preparing a film coated tablet comprising sacubitril sodium and valsartan disodium comprises the following steps;
- Sieving sacubitril sodium and talc and then mixing,
- Sieving microcrystalline cellulose, low-substituted hydroxypropylcellulose and crospovidone and then mixing,
- Adding valsartan disodium and mixing,
- Sieving silica colloidal anhydrous and then adding and mixing,
- Sieving magnesium stearate and then adding and mixing, and obtained intragranular composition,
- Compacting the intragranular composition,
- Sieving crospovidone and adding into the intragranular composition and then mixing,
- Sieving silica colloidal anhydrous and then adding and mixing,
- Sieving talc and then adding and mixing,
- Adding magnesium stearate and mixing,
- Compressing the total mixture, and obtained core tablets,
- Coating the core tablet with a film coating.

The formulation solves the sticking problem of the tablet in the preparation process. The preparation process also solves problems of degradation of the preparation due to hygroscopicity and improves stability of the tablet.

### Example 1:

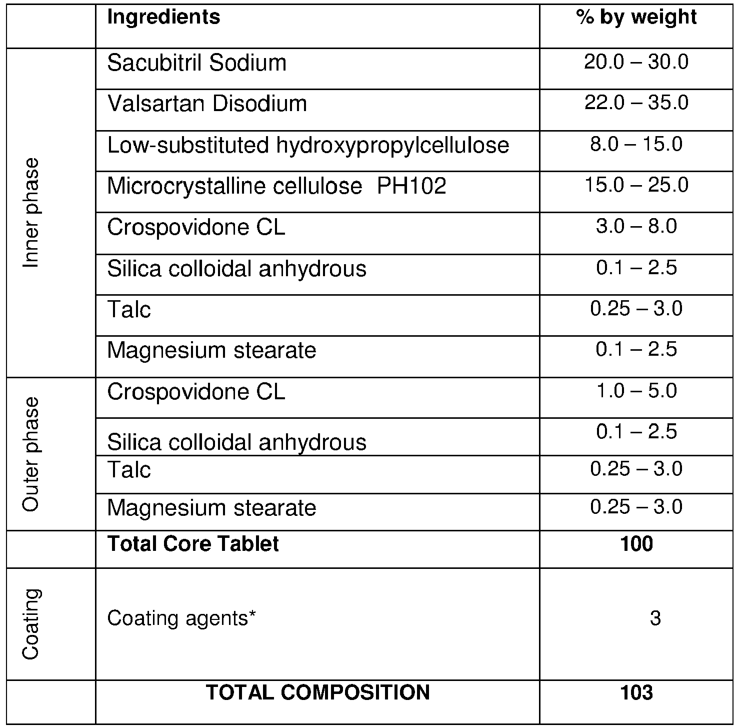

### Example 2:

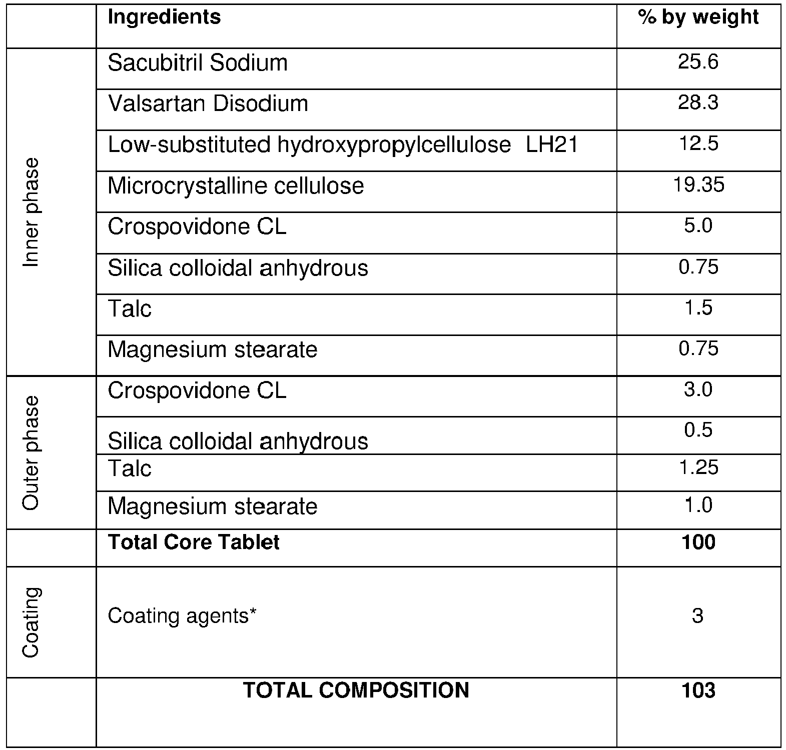

### Example 3:

| **Ingredients** | **Amount (% by weight of the total formulation)** |
|---|---|
| Sacubitril Sodium | 20.0 - 30.0 |
| Valsartan Disodium | 22.0 - 35.0 |
| Low-substituted hydroxypropylcellulose(L-HPC) | 8.0 - 15.0 |
| Microcrystalline cellulose | 15.0 - 25.0 |
| Crospovidone CL | 4.0 - 15.0 |
| Silica colloidal anhydrous | 0.2 - 5.0 |
| Talc | 0.5 - 6.0 |
| Magnesium stearate | 0.3 - 5.5 |
| **TOTAL CORE TABLET** | **100** |

### *Coating agents

- Hypromellose, substitution type 2910 (3 mPa·s)
- Titanium dioxide (E171)
- Macrogol (4000)
- Talc
- Iron oxide red (E172)
- Iron oxide black (E172)

### A process for example 1 or 2 or 3;

- Sieving sacubitril sodium and talc through 0.85 mm and then mixing,
- Sieving microcrystalline cellulose, low-substituted hydroxypropylcellulose and crospovidone through 0.85 mm and then mixing,
- Adding valsartan disodium and mixing,
- Sieving silica colloidal anhydrous and then adding and mixing,
- Sieving magnesium stearate and then adding and mixing, and obtained intragranular composition,
- Compacting the intragranular composition,
- Sieving crospovidone and adding into the intragranular composition and then mixing,
- Sieving silica colloidal anhydrous and then adding and mixing,
- Sieving talc and then adding and mixing,
- Adding magnesium stearate and mixing,
- Compressing the total mixture, and obtained core tablets,
- Coating the core tablet with a film coating.

## Claims

1. A film coated tablet comprising;
- an intragranular composition comprising sacubitril sodium, valsartan disodium and at least one disintegrant,
- an extragranular composition comprising at least one disintegrant,
wherein the amount of the disintegrants is 4.0% to 15.0% by weight of the total core tablet and the tablet is obtained by dry granulation.

2. The film coated tablet according to claim 1, wherein disintegrants are selected from the group comprising crospovidone, sodium starch glycolate, croscarmellose sodium, carboxymethyl cellulose, sodium alginate, corn starch, sodium glycine carbonate or mixtures thereof.

3. The film coated tablet according to claim 2, wherein the disintegrant is crospovidone.

4. The film coated tablet according to claim 1, wherein crospovidone is used both an intragranular composition and an extragranular composition.

5. The film coated tablet according to claim 1, wherein the amount of disintegrant is 5.0% to 10.0% by weight of the total core tablet.

6. The film coated tablet according to claim 1, wherein the dry granulation comprising the following steps;
- Preparing intragranular composition comprising sacubitril sodium, valsartan disodium and at least one pharmaceutically acceptable excipient,
- Preparing extragranular composition comprising at least one pharmaceutically acceptable excipient,
- Compressing the total mixture, and obtained core tablets,
- Coating the core tablet with a film coating,
wherein same excipients have been used for each composition.

7. The film coated tablet according to claim 1, wherein each composition further comprising the pharmaceutically acceptable excipients which are selected from the group comprising fillers, binders, disintegrants, lubricants, glidants or mixtures thereof.

8. The film coated tablet according to claim 7, wherein fillers are selected from the group comprising microcrystalline cellulose, pregelatinized starch, mannitol, dicalcium phosphate, lactose monohydrate, calcium carbonate, anhydrous lactose, calcium phosphate, calcium phosphate dehydrate, neutral pellets, cellulose acetate, ethylcellulose, polydextrose, polymethacrylates, sodium alginate, sodium chloride, sugar sphericals or mixtures thereof.

9. The film coated tablet according to claim 8, wherein the filler is microcrystalline cellulose.

10. The film coated tablet according to claim 7, wherein binders are selected from the group comprising low-substituted hydroxypropyl cellulose, hydroxypropyl cellulose, carboxymethylcellulose sodium, cellulose acetate phthalate, ethyl cellulose, glyceryl behenate, hydroxyethyl cellulose, hydroxyethylmethyl cellulose, hydroxypropyl starch, hypromellose, magnesium aluminum silicate, methylcellulose, polyethylene oxide, polymethacrylates, aluminum hydroxide, polyoxyethilene-alkyl ethers, pullulan or mixtures thereof.

11. The film coated tablet according to claim 10, wherein the binder is low-substituted hydroxypropyl cellulose.

12. The film coated tablet according to claim 7, wherein lubricants are selected from the group comprising talc, magnesium stearate, sodium stearyl fumarate, calcium stearate, zinc stearate, magnesium lauryl sulfate, sodium oleate, polyethylene glycol, sodium lauryl sulphate or mixtures thereof.

13. The film coated tablet according to claim 12, wherein the lubricant is magnesium stearate or talc or mixtures thereof.

14. The film coated tablet according to claim 1, wherein the film coated tablet comprises;
- Sacubitril Sodium
- Valsartan Disodium
- Low-substituted hydroxypropylcellulose
- Microcrystalline cellulose
- Crospovidone
- Silica colloidal anhydrous
- Magnesium stearate
- Talc

15. A process for preparing a film coated tablet comprising sacubitril sodium and valsartan disodium comprises the following steps;
- Sieving sacubitril sodium and at least one lubricant and then mixing,
- Sieving at least one filler, at least one binder and at least one disintegrant and then mixing,
- Adding valsartan disodium and mixing,
- Sieving at least one glidant and then adding and mixing,
- Sieving at least one lubricant and then adding and mixing, and obtained intragranular composition,
- Compacting the intragranular composition,
- Sieving at least one disintegrant and adding into the intragranular composition and then mixing,
- Sieving at least one glidant and then adding and mixing,
- Sieving at least one lubricant and then adding and mixing,
- Adding at least one lubricant and mixing,
- Compressing the total mixture, and obtained core tablets,
- Coating the core tablet with a film coating.
